# EUROPÄISCHE PATENTSCHRIFT

(11) **EP 0 685 456 B1**
(45) Veröffentlichungstag und Bekanntmachung des Hinweises auf die Patenterteilung: **15.04.1998**
(21) Anmeldenummer: 95107513.4
(22) Anmeldetag: 17.05.1995
(51) Int. Cl.: C07C 211/63, C07C 209/12

(54) **Bisquartäre Ammoniumverbindungen des 2,2'-Dimethyl-1,1'-binaphtyls und Verfahren zu deren Herstellung**
Bisquaternary ammonium derivatives of 2,2'-dimethyl-1,1'binaphtyl and process for their preparation
Dérivés d'ammonium bisquaternaires du diméthyl-2,2' binaphtyle-1,1' et procédé pour leur préparation

(30) Priorität: 26.05.1994 DE 4418349
(43) Veröffentlichungstag der Anmeldung: 06.12.1995
(73) Patentinhaber: HOECHST AKTIENGESELLSCHAFT, 65926 Frankfurt am Main (DE)
(72) Erfinder: Millauer, Hans, Dr., D-65760 Eschborn (DE)

(56) Entgegenhaltungen:
- EP-A- 0 630 884
- US-A- 2 744 902

## Beschreibung

Die Erfindung betrifft bisquartäre Ammoniumverbindungen des 2,2'-Dimethyl-1,1'-binaphthyls und Verfahren zu deren Herstellung.

Bisquartäre Ammoniumverbindungen des 2,2'-Dimethyl-1,1'-binaphthyls sind wertvolle Zwischenprodukte für die Herstellung von bis-tertiären Phosphinen.

Phosphine mit zwei tertiären Phosphin-Gruppen im Molekül, welche sperrige aromatische oder araliphatische Reste tragen, werden als zweizähnige Liganden zur Komplexierung von Schwermetallatomen wie Palladium oder Rhodium verwendet. Besonders zweizähnige Liganden, die sich strukturell vom 1,1'-Binaphthyl ableiten und in den Positionen 2 und 2' als Substituenten (Diphenylphosphino)-methyl-Gruppen tragen, bekannt unter dem Begriff "Naphos", bilden sehr stabile Komplexe, welche als Katalysatoren bei einer Reihe wichtiger industrieller Prozesse, z.B. Hydrierungen und Oxosynthesen eingesetzt werden.

Die am gleichen Tag eingereichte deutsche Patentanmeldung P 44 18 346.1 (EP-A-0 684 248) beschreibt ein sehr vorteilhaftes Verfahren zur Herstellung von tertiären Phosphinen, wofür als Ausgangsmaterialien die erfindungsgemäßen Ammoniumverbindungen benötigt werden.

Es bestand somit die Aufgabe derartige Verbindungen bereitzustellen.

Die Aufgabe wird gelöst durch Verbindungen der allgemeinen Formel (I) 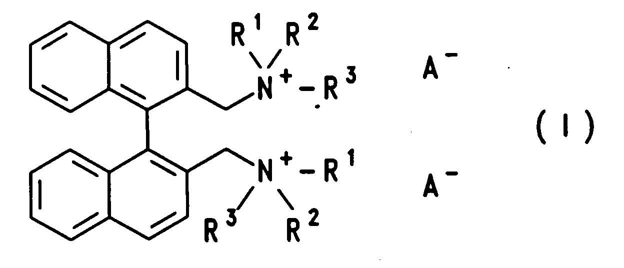 worin bedeuten:
R¹, R², R³ gleich oder verschieden (C₁-C₁₂)Alkyl, das in der Kette auch Sauerstoffatome oder N-R⁴-Glieder, worin R⁴ für (C₁-C₄)Alkyl steht, enthalten kann oder
R¹ (C₄-C₈)Cycloalkyl und R², R³ gleich oder verschieden (C₁-C₁₂)Alkyl oder
die beiden Reste R¹ zusammen eine Alkylenkette mit 2 bis 6 Kohlenstoffatomen und R², R³ gleich oder verschieden (C₁-C₁₂)Alkyl oder
die Reste R¹ und R², welche sich jeweils an demselben Stickstoffatom befinden, zusammen einen 5- oder 6-gliedrigen Ring, der in der Kette auch Sauerstoffatome oder N-R⁴-Glieder, worin R⁴ (C₁-C₄)Alkyl bedeutet, enthalten kann und der Rest R³ einen Alkylrest mit 1 bis 12 Kohlenstoffatomen oder
die Reste R¹, R², R³, welche sich jeweils an demselben Stickstoffatom befinden, zusammen ein bicyclisches Ringsystem mit einem Stickstoffatom als Brückenkopfatom und gegebenenfalls weiteren Sauerstoffatomen oder N-R⁴-Gliedern, worin R⁴ (C₁-C₄)Alkyl bedeutet, im Ringsystem und
A ein Chlor-, Brom-, Iodatom oder ein OSO₃R⁵-Rest, worin R⁵ für (C₁-C₄)Alkyl oder Aryl steht.

Von großem Interesse sind beispielsweise die folgenden Verbindungen der Formel (I):
2,2'-Bis-[(trimethylammonio)-methyl]-1,1'-binaphthyl-Salze
2,2'-Bis-[(triethylammonio)-methyl]-1,1'-binaphthyl-Salze
2,2'-Bis-[(tripropylammonio)-methyl]-1,1'-binaphthyl-Salze
2,2'-Bis-[(tributylammonio)-methyl]-1,1'-binaphthyl-Salze
2,2'-Bis-[(trihexylammonio)-methyl]-1,1'-binaphthyl-Salze
2,2'-Bis-[(trioctylammonio)-methyl]-1,1'-binaphthyl-Salze
2,2'-Bis-[(N-ethyldimethylammonio)-methyl]-1,1'-binaphthyl-Salze
2,2'-Bis-[(N,N-dimethylbutylammonio)-methyl]-1,1'-binaphthyl-Salze
2,2'-Bis-[(N,N-dimethylcyclohexylammonio)-methyl]-1,1'-binaphthyl-Salze
2,2'-Bis-[(N,N-dicyclohexylmethylammonio)-methyl]-1,1'-binaphthyl-Salze
2,2'-Bis-[(N-methylpiperidinio)-methyl]-1,1'-binaphthyl-Salze
2,2'-Bis-[(N,N'-dimethylpiperazinio)-methyl]-1,1'-binaphthyl-Salze
2,2'-Bis-[(N-methyl-morpholinio)-methyl]-1,1'-binaphthyl-Salze

Besonders wichtig sind auch: 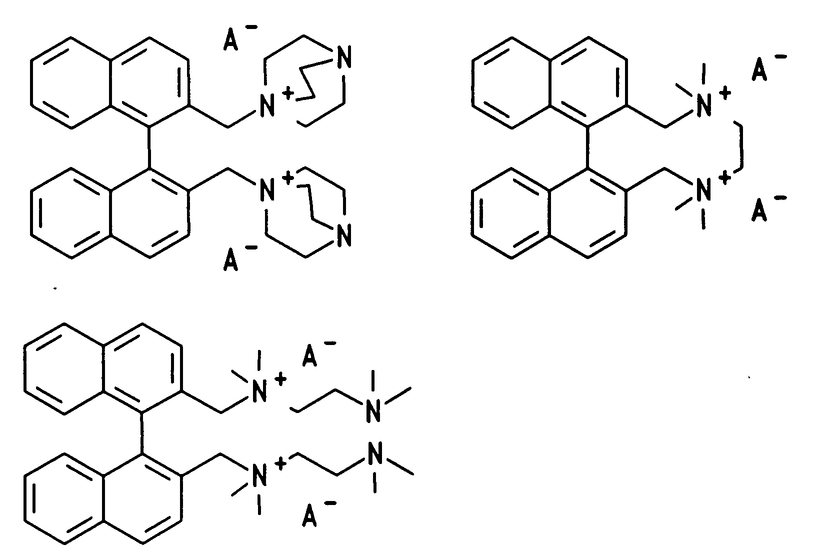 Die Erfindung betrifft ferner ein Verfahren zur Herstellung der Verbindungen der Formel (I), dadurch gekennzeichnet, daß man Verbindungen der Formel (II) 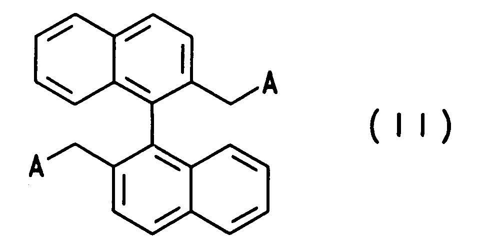 worin A die vorstehend genannte Bedeutung besitzt,
bei Temperaturen von 20 bis 150°C in Gegenwart eines Lösungsmittels, gegebenenfalls unter Druck,
mit einer Stickstoffbase der Formel (III) 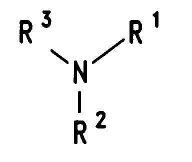 worin R¹, R² und R³ die vorstehend genannte Bedeutung besitzen, umsetzt.

Da die erfindungsgemäßen Produkte gemäß Formel (I) in vielen Fällen dazu neigen, mit dem benutzten Lösemittel stabile Solvat-Komplexe zu bilden, ist bei der Wahl des Lösemittels dessen Unschädlichkeit für die nachfolgende Reaktionsstufe zu berücksichtigen. Geeignete Lösemittel sind zum Beispiel Kohlenwasserstoffe, Ether, halogenierte Kohlenwasserstoffe, Ketone, Nitroverbindungen oder Nitrile.
In vielen Fällen bewährt haben sich z.B.: Isohexan, n-Heptan, Cyclohexan, Schnitte von Kohlenwasserstoffen (Benzine) mit Siedepunktsbereichen zwischen 70 und 250°C, Toluol, Xylol, Methyl-t-butylether, Diisopropylether, Tetrahydrofuran, Chloroform, Chlorbenzol, Aceton, Methyl-i-butylketon, Nitromethan, Acetonitril, Dimethylformamid und Dimethylacetamid; bevorzugt sind Chlorbenzol und Dimethylformamid.

Die Reaktionstemperaturen betragen vorzugsweise 50 bis 120°C. Bei der Verwendung von leicht flüchtigen Aminen ist die Anwendung eines erhöhten Druckes nützlich. Der erforderliche Druckbereich liegt zwischen 1 und etwa 10 bar. Die Reaktionsdauer beträgt zwischen 1 und 12 Stunden.

Das Reaktionsgemisch wird zweckmäßigerweise während der Umsetzung ständig gerührt. Die erfindungsgemäßen Produkte gemäß Formel (I) sind in einem Teil der verwendeten Lösemittel schwerlöslich und fallen bereits während der Umsetzung aus. Die Abtrennung der Produkte erfolgt in diesem Fall durch Filtration. Liegen lösliche Verfahrensprodukte vor, so kann die Isolierung nach Abdestillieren des Lösemittels und gegebenenfalls des überschüssigen Amins erfolgen.
Bei der Verwendung bestimmter Lösemittel wie beispielsweise von Dimethylformamid, Dimethylacetamid, Acetonitril oder Tetrahydrofuran, kann auf die Isolierung des Produkts verzichtet werden; in diesem Fall kann das Reaktionsgemisch direkt in der nachfolgenden Reaktionsstufe eingesetzt werden.

Die zur Herstellung des erfindungsgemäßen Produkte gemäß Formel (I) benötigten Ausgangsverbindungen gemäß Formel (II) sind beispielsweise durch Seitenkettenbromierung von 2,2'-Dimethyl-1,1'-binaphthyl nach bekannten Verfahren zugänglich. Die als Ausgangsverbindungen gemäß Formel (III) benötigten tertiären Amine sind bekannt und größtenteils kommerziell verfügbar; ihre Herstellung erfolgt nach bekannten Verfahren.

### Beispiel 1

In einem Rührkolben von 100 ml Inhalt mit einem auf -20°C kühlbaren Rückflußkühler, Thermometer und Heizbad werden 11,0 g (0,025 Mol) 2,2'-Bis-(brommethyl)-1,1'-binaphthyl, 4,43 g (0,075 Mol) Trimethylamin in 50 ml Dimethylformamid vorgelegt und unter Rühren allmählich auf 70°C erwärmt. Man rührt noch 3 Stunden bei dieser Temperatur. Nach Abkühlen auf Raumtemperatur saugt man das ausgefallene Produkt ab, wäscht mit wenig kaltem Dimethylformamid nach und trocknet im Vakuumexsiccator über konz. Schwefelsäure. Die Ausbeute beträgt 11,8 g (74 % der Theorie) 2,2'-Bis-[(trimethylammonio)-methyl]-1,1'-binaphthyl-dibromid; das Produkt enthält nach dem Trocknen noch ein Mol Dimethylformamid.
¹H-NMR (300 MHz, D₂O) δ in ppm, referenziert auf D₂O:
   7,2 - 8,2 (12 H, Aryl-H)
   4,7 (2 H, Aryl-CH^{A}H^{B}-N⁺)
   3,9 (2 H, Aryl-CH^{A}H^{B}-N⁺, J_{AB} = 15 Hz)
   2,6 (18 H, -N⁺ (CH₃)₃)

## Patentansprüche

1. Bisquartäre Ammoniumverbindungen der Formel (I) 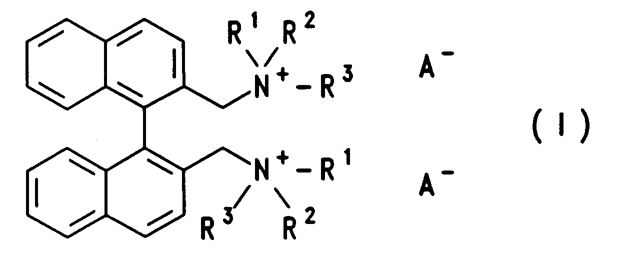 worin bedeuten:
R¹, R², R³ gleich oder verschieden (C₁-C₁₂)Alkyl, das in der Kette auch Sauerstoffatome oder N-R⁴-Glieder, worin R⁴ für (C₁-C₄)Alkyl steht, enthalten kann oder
R¹ (C₄-C₈)Cycloalkyl und R², R³ gleich oder verschieden (C₁-C₁₂)Alkyl oder
die beiden Reste R¹ zusammen eine Alkylenkette mit 2 bis 6 Kohlenstoffatomen und R², R³ gleich oder verschieden (C₁-C₁₂)Alkyl oder
die Reste R¹ und R², welche sich jeweils an demselben Stickstoffatom befinden, zusammen einen 5- oder 6-gliedrigen Ring, der in der Kette auch Sauerstoffatome oder N-R⁴-Glieder, worin R⁴ (C₁-C₄)Alkyl bedeutet, enthalten kann und der Rest R³ einen Alkylrest mit 1 bis 12 Kohlenstoffatomen oder
die Reste R¹, R², R³, welche sich jeweils an demselben Stickstoffatom befinden, zusammen ein bicyclisches Ringsystem mit einem Stickstoffatom als Brückenkopfatom und gegebenenfalls weiteren Sauerstoffatomen oder N-R⁴-Gliedern, worin R⁴ (C₁-C₄)Alkyl bedeutet, im Ringsystem und
A ein Chlor-, Brom-, Iodatom oder ein OSO₃R⁵-Rest, worin R⁵ für (C₁-C₄)Alkyl oder Aryl steht.

2. Verbindungen der Formel (I) aus den Gruppen:
2,2'-Bis-[(trimethylammonio)-methyl]-1,1'-binaphthyl-Salze
2,2'-Bis-[(triethylammonio)-methyl]-1,1'-binaphthyl-Salze
2,2'-Bis-[(tripropylammonio)-methyl]-1,1'-binaphthyl-Salze
2,2'-Bis-[(tributylammonio)-methyl]-1,1'-binaphthyl-Salze
2,2'-Bis-[(trihexylammonio)-methyl]-1,1'-binaphthyl-Salze
2,2'-Bis-[(trioctylammonio)-methyl]-1,1'-binaphthyl-Salze
2,2'-Bis-[(N-ethyldimethylammonio)-methyl]-1,1'-binaphthyl-Salze
2,2'-Bis-[(N,N-dimethylbutylammonio)-methyl]-1,1'-binaphthyl-Salze
2,2'-Bis-[(N,N-dimethylcyclohexylammonio)-methyl]-1,1'-binaphthyl-Salze
2,2'-Bis-[(N,N-dicyclohexylmethylammonio)-methyl]-1,1'-binaphthyl-Salze
2,2'-Bis-[(N-methylpiperidinio)-methyl]-1,1'-binaphthyl-Salze
2,2'-Bis-[(N,N'-dimethylpiperazinio)-methyl]-1,1'-binaphthyl-Salze
2,2'-Bis-[(N-methyl-morpholinio)-methyl]-1,1'-binaphthyl-Salze

3. Verbindungen der Formel (I) aus den Gruppen:

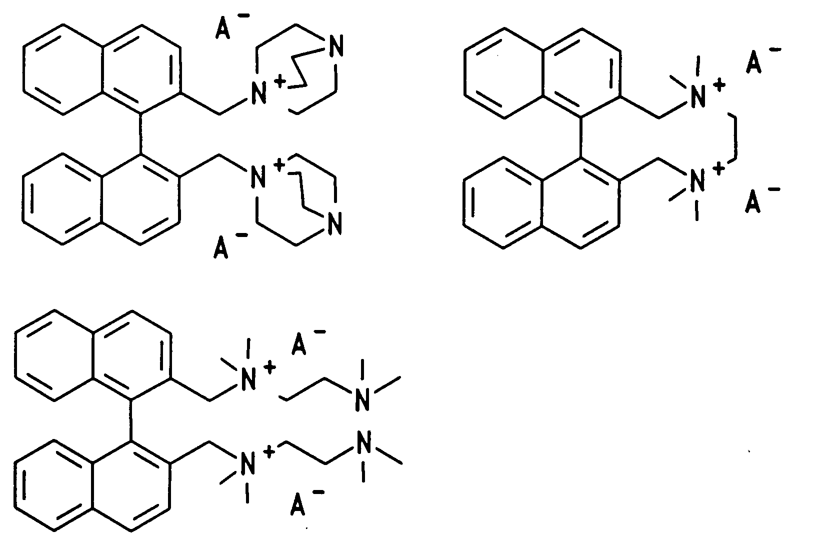

4. Verfahren zur Herstellung der Verbindungen der Formel (I), dadurch gekennzeichnet, daß man Verbindungen der Formel (II) 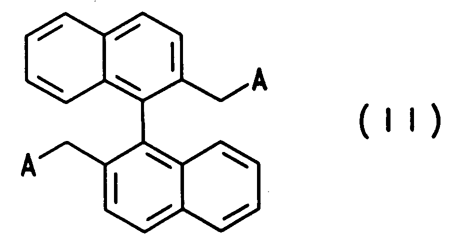 worin A die vorstehend genannte Bedeutung besitzt,
bei Temperaturen von 20 bis 150°C in Gegenwart eines Lösungsmittels, gegebenenfalls unter Druck,
mit einer Stickstoffbase der Formel (III) 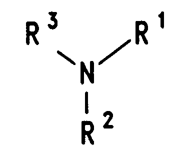 worin R¹, R² und R³ die vorstehend genannte Bedeutung besitzen, umsetzt.

5. Verfahren nach Anspruch 4, dadurch gekennzeichnet, daß als Lösungsmittel Kohlenwasserstoffe, Ether, halogenierte Kohlenwasserstoffe, Ketone, Nitroverbindungen oder Nitrile eingesetzt werden.

6. Verfahren nach Anspruch 4, dadurch gekennzeichnet, daß als Lösungsmittel Isohexan, n-Heptan, Cyclohexan, Schnitte von Kohlenwasserstoffen (Benzine) mit Siedepunktsbereichen zwischen 70 und 250°C, Toluol, Xylol, Methyl-t-butylether, Diisopropylether, Tetrahydrofuran, Chloroform, Chlorbenzol, Aceton, Methyl-i-butylketon, Nitromethan, Acetonitril, Dimethylformamid oder Dimethylacetamid eingesetzt werden.

7. Verfahren nach Anspruch 4, dadurch gekennzeichnet, daß als Lösungsmittel Chlorbenzol oder Dimethylformamid eingesetzt werden.

8. Verfahren nach einem der Ansprüche 4 bis 7, dadurch gekennzeichnet, daß bei Temperaturen von 50 bis 120°C umgesetzt wird.

9. Verfahren nach einem der Ansprüche 4 bis 8, dadurch gekennzeichnet, daß bei einem Druck von 1 bis 10 bar umgesetzt wird.

## Claims

1. A bisquaternary ammonium compound of the formula (I) 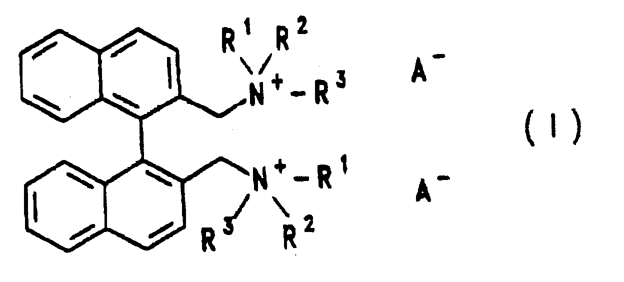 where:
R¹, R², R³ are identical or different and are (C₁-C₁₂)alkyl which can also contain oxygen atoms or N-R⁴ moieties, where R⁴ is (C₁-C₄)alkyl, in the chain or
R¹ is (C₄-C₈)cycloalkyl and R², R³ are identical or different and are (C₁-C₁₂)alkyl or
the two radicals R¹ together form an alkylene chain having from 2 to 6 carbon atoms and R², R³ are identical or different and are (C₁-C₁₂)alkyl or
the radicals R¹ and R² located on the same nitrogen atom together form a 5-membered or 6-membered ring, which can also contain oxygen atoms or N-R⁴ moieties, where R⁴ is (C₁-C₄)alkyl, in the chain and the radical R³ is an alkyl radical having from 1 to 12 carbon atoms or
the radicals R¹, R², R³ located on the same nitrogen atom together form a bicyclic ring system having a nitrogen atom as bridge head atom and, if desired, further oxygen atoms or N-R⁴ moieties, where R⁴ is (C₁-C₄)alkyl, in the ring system and
A is a chlorine, bromine, iodine atom or a OSO₃R⁵ radical, where R⁵ is (C₁-C₄)alkyl or aryl.

2. A compound of the formula (I) from the groups consisting of:
2,2'-bis[(trimethylammonio)methyl]-1,1'-binaphthyl salts
2,2'-bis[(triethylammonio)methyl]-1,1'-binaphthyl salts
2,2'-bis[(tripropylammonio)methyl]-1,1'-binaphthyl salts
2,2'-bis[(tributylammanio)methyl]-1,1'-binaphthyl salts
2,2'-bis[(trihexylammonio)methyl]-1,1'-binaphthyl salts
2,2'-bis[(trioctylammonio)methyl]-1,1'-binaphthyl salts
2,2'-bis[(N-ethyldimethylammonio)methyl]-1,1'-binaphthyl salts
2,2'-bis[(N,N-dimethylbutylammonio)methyl]-1,1'-binaphthyl salts
2,2'-bis[(N,N-dimethylcyclohexylammonio)methyl]-1,1'-binaphthyl salts
2,2'-bis[(N,N-dicyclohexylmethylammonio)methyl]-1,1'-binaphthyl salts
2,2'-bis[(N-methylpiperidinio)methyl]-1,1'-binaphthyl salts
2,2'-bis[(N,N'-dimethylpiperazinio)methyl]-1,1'-binaphthyl salts
2,2'-bis[(N-methylmorpholinio)methyl]-1,1'-binaphthyl salts.

3. A compound of the formula (I) from the groups consisting of:

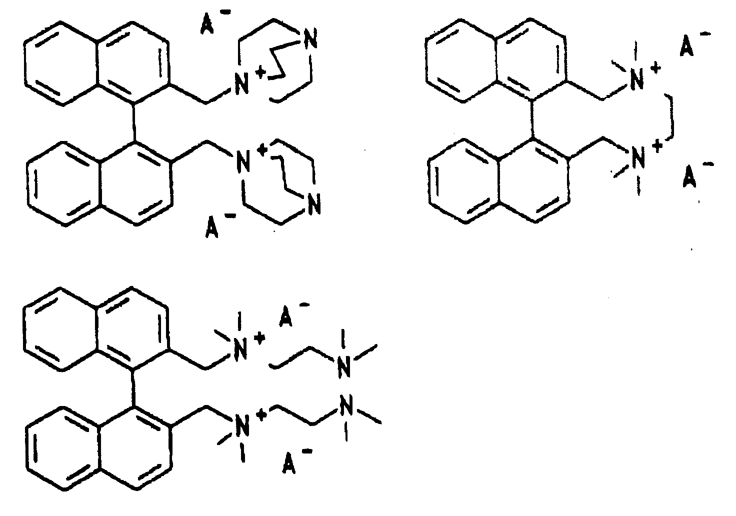

4. A process for preparing the compounds of the formula (I), which comprises reacting compounds of the formula (II) 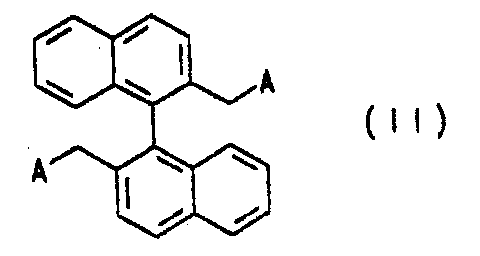 where A is as defined above,
at temperatures of from 20 to 150°C in the presence of a solvent, at atmospheric or superatmospheric pressure,
with a nitrogen base of the formula (III) 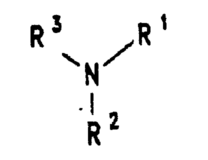 where R¹, R² and R³ are as defined above.

5. The process as claimed in claim 4, wherein hydrocarbons, ethers, halogenated hydrocarbons, ketones, nitro compounds or nitriles are used as solvent.

6. The process as claimed in claim 4, wherein iso-hexane, n-heptane, cyclohexane, hydrocarbon fractions (petroleum spirit) having boiling point ranges between 70 and 250°C, toluene, xylene, methyl t-butyl ether, diisopropyl ether, tetrahydrofuran, chloroform, chlorobenzene, acetone, methyl i-butyl ketone, nitromethane, acetonitrile, dimethylformamide or dimethylacetamide are used as solvent.

7. The process as claimed in claim 4, wherein chlorobenzene or dimethylformamide are used as solvent.

8. The process as claimed in any one of claims 4 to 7, wherein the reaction is carried out at temperatures of from 50 to 120°C.

9. The process as claimed in any one of claims 4 to 8, wherein the reaction is carried out at a pressure of from 1 to 10 bar.

## Revendications

1. Dérivés d'ammonium bisquaternaires de Formule ( 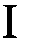 ) 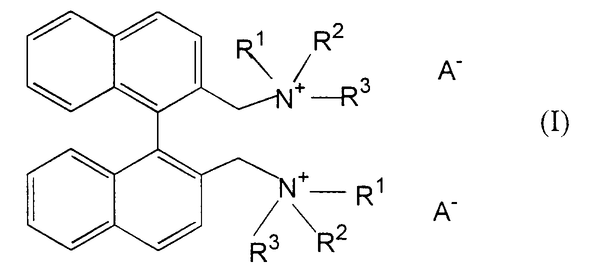 dans laquelle:
R¹, R², R³ sont semblables ou différents et représentent un groupe alkyle en (C₁-C₁₂), qui peut contenir aussi dans la chaîne un atome d'oxygène ou un reste N-R⁴, dans lequel R⁴ représente un groupe alkyle en (C₁-C₄), ou
R¹ est un groupe cycloalkyle en (C₄-C₈) et R², R³ sont semblables ou différents et représentent un groupe alkyle en (C₁-C₁₂), ou
les deux restes R¹ forment ensemble une chaîne alkyle de 2 à 6 atomes de carbone et R², R³ sont semblables ou différents et représentent un groupe alkyle en (C₁-C₁₂), ou
les restes R¹ et R², qui se trouvent respectivement sur le même atome d'azote, forment ensemble un noyau à 5 ou 6 membres, qui peut contenir aussi dans la chaîne un atome d'oxygène ou un reste N-R⁴, dans lequel R⁴ représente un groupe alkyle en (C₁-C₄) et le reste R³ signifie un reste alkyle avec de 1 à 12 atomes de carbone ou
les restes R¹, R², R³, qui se trouvent respectivement sur le même atome d'azote, forment ensemble un système de noyau bicyclique avec un atome d'azote comme atome tête de pont et éventuellement, dans le système de noyau, des autres atomes d'oxygène ou des restes N-R⁴, dans lesquels R⁴ représente un groupe alkyle en (C₁-C₄), et
A signifie un atome de chlore, brome ou iode ou un reste OSO₃R⁵, dans lequel R⁵ est un radical alkyle en (C₁-C₄) ou aryle.

2. Composés de Formule ( 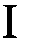 ) choisis dans les groupes:
des sels de bis[(triméthylammonio)-méthyl]-2,2' binaphtyle-1,1'
des sels de bis[(triéthylammonio)-méthyl]-2,2' binaphtyle-1,1'
des sels de bis[(tripropylammonio)-méthyl]-2,2' binaphtyle-1,1'
des sels de bis[(tributylammonio)-méthyl]-2,2' binaphtyle-1,1'
de sels de bis[(trihexylammonio)-méthyl[-2,2' binaphtyle-1,1'
des sels de bis[(trioctylammonio)-méthyl]-2,2' binaphtyle-1,1'
des sels de bis[(N-éthyldiméthylammonio)-méthyl]-2,2' binaphtyle-1,1'
des sels de bis[(N,N-diméthylbutylammonio)-méthyl]-2,2' binaphtyle-1,1'
des sels de bis[(N,N-diméthylcyclohexylammonio)-méthyl]-2,2' binaphtyle-1,1'
des sels de bis[(N,N-dicyclohexylméthylammonio)-méthyl]-2,2' binaphtyle-1,1'
des sels de bis[(N-méthylpipéridinio)-méthyl]-2,2' binaphtyle-1,1'
des sels de bis[(N-N'-diméthylpipérazinio)-méthyl]-2,2' binaphtyle-1,1'
des sels de bis[(N-méthyl-morpholino)-méthyl]-2,2' binaphtyle-1,1'

3. Composés de Formule ( 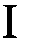 ) choisis dans les groupes:

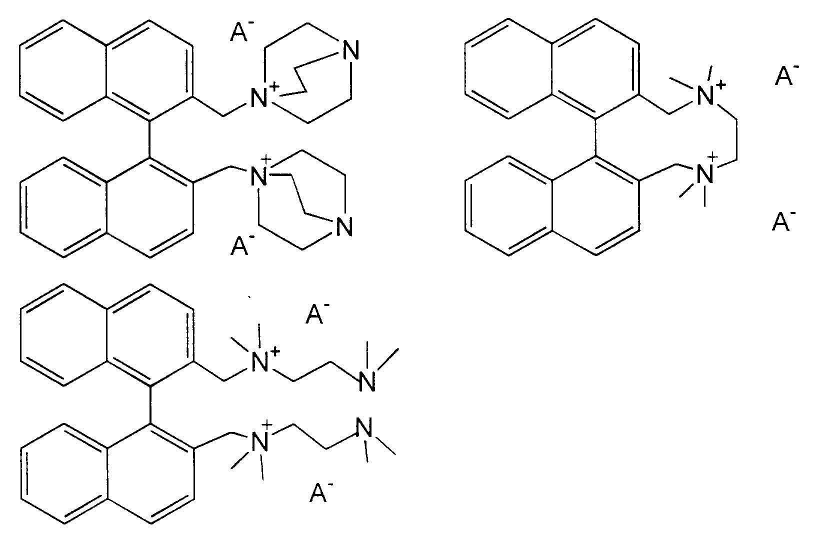

4. Procédé de préparation des composés de Formule ( 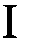 ), caractérisé en ce que l'on fait réagir un composé de Formule ( 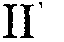 ) 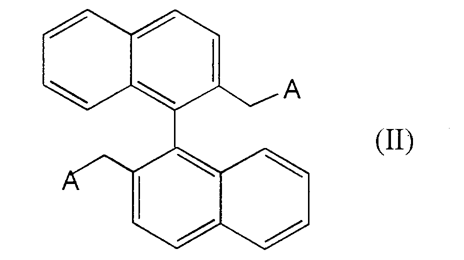 dans laquelle A a la signification mentionnée précédemment,
à des températures de 20 à 150 °C en présence d'un solvant, éventuellement sous pression,
avec une base azotée de Formule (( 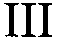 ) 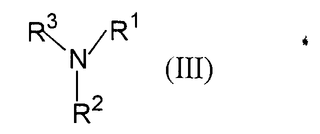 dans laquelle R¹, R² et R³ ont la signification mentionnée précédemment.

5. Procédé selon la revendication 4, caractérisé en ce qu'on utilise comme solvant des hydrocarbures, de l'éther, des hydrocarbures halogénés, des cétones, des composés nitro ou des nitriles.

6. Procédé selon la revendication 4, caractérisé en ce qu'on utilise comme solvant l'isohexane, le n-heptane, le cyclohexane, des coupes d'hydrocarbures (essence) avec des domaines de point d'ébullition entre 70 et 250 °C, le toluène, le xylène, l'éther méthyl t-butylique, l'éther diisopropylique, le tétrahydrofurane, le chloroforme, le chlorobenzène, l'acétone, la méthyl-i-butylcétone, le nitrométhane, l'acétonitrile, le diméthylformamide ou le diméthylacétamide.

7. Procédé selon la revendication 4, caractérisé en ce qu'on utilise comme solvant le chlorobenzène ou le diméthylformamide.

8. Procédé selon l'une des revendications 4 à 7, caractérisé en ce qu'on effectue la réaction à des températures de 50 à 120°C.

9. Procédé selon l'une des revendications 4 à 8, caractérisé en ce qu'on effectue la réaction à une pression de 1 à 10 bar.
